# EUROPEAN PATENT SPECIFICATION

(11) **EP 1 865 057 B1**
(45) Date of publication and mention of the grant of the patent: **16.06.2010**
(21) Application number: 06714782.7
(22) Date of filing: 27.02.2006
(51) Int. Cl.: C12N 15/09, C12M 1/00, C12Q 1/68, G01N 33/53, G01N 37/00

(54) **DNA ARRAY AND METHOD FOR ANALYSIS OF GENE EXPRESSION IN BREWING YEAST**
DNA-ARRAY UND VERFAHREN ZUR ANALYSE DER GENEXPRESSION IN BIERHEFE
RÉSEAU D'ADN ET PROCÉDÉ D'ANALYSE DE L'EXPRESSION DE GÈNES DE LEVURE DE BIÈRE

(30) Priority: 03.03.2005 JP 2005059717
(43) Date of publication of application: 12.12.2007
(73) Proprietor: Sapporo Breweries Limited, Tokyo 150-8522 (JP)
(72) Inventor: SATO, Masahide, c/o Sapporo Breweries Limited, Yaizu-shi, Shizuoka, 425-0013 (JP); KOBAYASHI, Naoyuki, c/o Sapporo Breweries Limited, Yaizu-shi, Shizuoka, 425-0013 (JP); FUKUHARA, Syunsuke, c/o Sapporo Breweries Limited, Hita-shi, Oita 877-0054 (JP); YOKOI, Shigehisa, c/o Sapporo Breweries Limited, Hita-shi, Oita 877-0054 (JP)
(74) Representative: Grünecker, Kinkeldey, Stockmair & Schwanhäusser Anwaltssozietät
(86) International application number: PCT/JP2006/303645
(87) International publication number: WO 2006/093103

(56) References cited:
- WO-A-2004/013277
- JP-A- 2004 283 169
- TRENKLE T. ET AL.: 'Non-stoichiometric reduced complexity probes for cDNA assays' NUCLEIC ACIDS RESEARCH vol. 26, no. 17, 1998, pages 3883 - 3891, XP002122209
- JAMES T.C. ET AL.: 'Comparative Analysis of Global Gene Expression in Lager and laboratory Yeast Strains Grown in Wort' PROCEEDINGS OF THE IEEE vol. 90, no. 12, 2002, pages 1887 - 1899, XP003001317
- JAMES T.C. ET AL.: 'Transcription profile of brewery yeast under fermentation conditions' J. APPLIED MICROBIOL. vol. 94, 2003, pages 432 - 448, XP003001318
- SATO M. ET AL.: 'Genome DNA Danpen o Koteika Shita DNA Microarray ni yoru Shitamen Beer Kobo no Hatsugen kaiseki' JAPAN SOCIETY FOR BIOSCIENCE, BIOTECHNOLOGY, AND AGROCHEMISTRY 2005 NENDO TAIKAI KOEN YOSHISHU 05 March 2005, page 247, XP003001321
- KONDO H. ET AL.: 'Gene expression analysis of brewing yeasts during beer fermentations using DNA microarray' PROCEEDINGS OF THE CONGRESS EUROPEAN BREWERY CONVENTION,29TH 2003, pages 49/1 - 49/13, XP003001322
- NAKAO Y. ET AL.: 'Whole genome sequence of a lager brewing yeast' PROCEEDINGS OF THE CONGRESS-EUROPEAN BREWERY CONVENTION,29TH 2003, pages 48/1 - 48/7, XP003001323
- PENG X. ET AL.: 'Patterns of yeast gene expression during fermentation and in response to stress' PROCEEDINGS OF THE CONGRESS-EUROPEAN BREWERY CONVENTION,29TH 2003, pages 45/1 - 45/13, XP003001324
- RAY A. ET AL.: 'Negative Substraction Hybridization: An efficient method to isolate large numbers of condition-specific cDNAs' BMC GENOMICS vol. 5, 2004, pages 1 - 11, XP003001325

## Description

### Technical Field

The present invention relates to a DNA array and to a method for analysis of brewer's yeast gene expression.

### Background Art

Bottom-fermenting yeast is the most widely used yeast for brewing of alcoholic beverages such as beer. In recent years, the use of DNA arrays has been introduced as a technique for analysis of gene expression of bottom-fermenting yeast (Non-patent document 1). [Non-patent document 1] FEMS Yeast Research, Vol. 2, 563-573(2002).

The document WO 2004/013277 discloses an array of single stranded large circular molecules for expression profiling of diseased tissue.

The document, Rappel et al, Proceedings of the IEEE, vol. 90, p. 1887-1899, (2003), discloses global gene expression profiling of brewer's yeast and uses a cDNA microarray containing 6300 ORFs.

The document JP2004283169 discloses arrays containing genomic sequences from brewery yeast. In particular, arrays produced with DNasel fragments and oligonucleotide arrays are disclosed.

### Disclosure of the Invention

### Problems to be Solved by the Invention

Since the genomic sequence data for bottom-fermenting yeast has not yet been made public, the technique disclosed in Non-patent document 1 is not sufficient to be used for controlling the bottom-fermenting yeast fermentation process, as it is not clear which gene expression was analyzed.

On the other hand, because bottom-fermenting yeast has a large genome size (24-26M bp) and a complex chromosome structure that also includes non-*Saccharomyces cerevisiae* genes, acquisition of its genomic sequence data has required prolonged time and high cost.

It is an object of the present invention to provide a gene expression analysis method that can be satisfactorily used to control the brewer's yeast fermentation process without requiring previous acquisition of genomic sequence data of brewer's yeast, as well as a DNA array used in the gene expression analysis method.

### Means for Solving the Problems

In order to achieve the object stated above, the invention provides a DNA array comprising plasmids containing genomic DNA fragments of brewer's yeast bound onto a plurality of spots on a substrate.

The invention further provides a gene expression analysis method for brewer's yeast, comprising
(a) a step of extracting total RNA from two kinds of brewer's yeasts of different strains or states,
(b) a step of obtaining cDNA or cRNA labeled with a first fluorescent dye from the total RNA of one kind and obtaining cDNA or cRNA labeled with a second fluorescent dye from the total RNA of the other kind (hereinafter, cDNA labeled with a fluorescent dye will also be referred to as "labeled cDNA", and cRNA labeled with a fluorescent dye will also be referred to as "labeled cRNA"),
(c) a step of competitively hybridizing the two different labeled cDNAs or two different labeled cRNAs on a DNA array comprising plasmids containing genomic DNA fragments of brewer's yeast, either identical to or different from the brewer's yeast, bound onto a plurality of spots on a substrate,
(d) a step of measuring the fluorescence emitted by the first and second fluorescent dyes at each spot,
(e) a step of comparing the intensity of fluorescence emitted by the first fluorescent dye with the intensity of fluorescence emitted by the second fluorescent dye, and
(f) a step of analyzing the sequences of the genomic DNA fragments in the plasmids at spots where a difference in fluorescent intensity is observed.

In the gene expression analysis method using the DNA array of the invention, gene expression status is compared among a plurality of yeasts of different strains or states, and sequence analysis is performed only for genes in which a difference is observed so that it is not necessary to acquire brewer's yeast genomic sequence data beforehand. Whereas the relationship between differences in yeast strain and state and differences in gene expression status can be determined so that the method may be satisfactorily utilized for control of the brewer's yeast fermentation process. Expression status can also be analyzed for nucleotide sequence regions whose analysis is not possible by ordinary analysis programs used to predict exon regions and open reading frames. Such nucleotide sequence regions include non-coding regions (regions that do not code for amino acid sequences) that are not the target of analysis by the aforementioned programs, as well as some open reading frames that cannot be analyzed due to restrictions on the abilities of the programs.

Preferred genomic DNA fragments of brewer's yeast to be contained in the plasmids of the DNA array are those derived from bottom-fermenting yeast, most widely used for beer brewing.

The DNA array preferably further comprises another spot binding a brewer's yeast gene of known function (hereinafter referred to as "known gene").

Using such a DNA array is capable of identifying a gene present in a DNA fragment and estimating its function by analysing the sequence of a genomic DNA fragment at a spot exhibiting a pattern of fluorescence intensity (proportion of fluorescence intensity between the first and second fluorescent dye) similar to or in synchrony with that of the spot binding the known gene. For example, when the genomic DNA includes a gene that is functionally similar to a known gene important for brewing but that differs in its nucleotide sequence from the known gene, the gene can be identified by analysing the sequence of the genomic DNA fragment similar to or in synchrony with the known gene in their differences in fluorescence intensity pattern, i.e. the status of gene expression of the two different yeasts. In other words, it is possible to discover a known or new gene whose expression pattern during fermentation is in synchrony with the known gene that is important for brewing and is functionally similar thereto, but has a different gene sequence or amino acid sequence.

When the known gene is constitutively expressed regardless of the strain or state of the brewer's yeast, a DNA array having the construction described above is also capable of easily evaluating the performance of hybridization experiments at spots surrounding it (for example, in a block containing the spot), based on the fluorescence intensity at the spot of the known gene.

As a suitable type of DNA array also comprising another spot binding the known gene, there may be used one wherein blocks consisting of a plurality of spots binding plasmids including brewer's yeast genomic DNA fragments and a spot binding the known gene, are arranged on a substrate. By using this type of DNA array, it is possible to estimate functions of genes in genomic DNA fragments, or evaluate the performance of hybridization experiments, in each block with respect to the known gene. The general type referred to above also includes a type wherein the blocks have equal shapes and spots binding the known gene are present at the same position of each block, which is particularly preferred since it facilitates screening of the positions of spots binding the plasmid or known gene.

The competitive hybridization in step (c) above is conducted in a competitive manner on a single DNA array, but instead of competitive hybridization as in step (c), hybridization may be carried out on separate DNA arrays for different yeast strains or states (providing that the same plasmids or known gene are bound at the same positions of each DNA array). The invention still further provides a gene expression analysis method for brewer's yeast, comprising
(a) a step of extracting total RNA from two kinds of brewer's yeasts of different strains or states,
(b) a step of obtaining the following (b1) or (b2) from the total RNA:
   (b1) first labeled cDNA labeled with a first fluorescent dye and second labeled cDNA labeled with a second fluorescent dye
   (b2) first labeled cRNA labeled with a first fluorescent dye and second labeled cRNA labeled with a second fluorescent dye,
(c) a step of preparing two DNA arrays binding plasmids containing genomic DNA fragments of brewer's yeast identical to or different from the brewer's yeast at a plurality of spots on a substrate (where the two DNA arrays are identical and one is designated as the first DNA array while the other is designated as the second DNA array), and hybridizing them in a manner according to (c1) or (c2) below:
   (c1) first labeled cDNA on the first DNA array and second labeled cDNA on the second DNA array
   (c2) first labeled cRNA on the first DNA array and second labeled cRNA on the second DNA array,
(d) a step of measuring the fluorescence emitted by the first fluorescent dye and second fluorescent dye at each corresponding spot of first DNA array and second DNA array,
(e) a step of comparing the intensity of fluorescence emitted by the first fluorescent dye with the intensity of fluorescence emitted by the second fluorescent dye, and
(f) a step of analyzing the sequences of the genomic DNA fragments in the plasmids at spots where a difference in fluorescent intensity is observed. Here, the first fluorescent dye and second fluorescent dye may be differing dyes or identical dyes.

### Brief Description of the Drawings

Fig. 1 is a plan view schematically showing a DNA array according to a first embodiment.
Fig. 2 is a plan view schematically showing a DNA array according to a second embodiment.
Fig. 3 is a plan view schematically showing the DNA array prepared in Example 1.
Fig. 4 is a plan view schematically showing a magnified view of one block of the DNA array prepared in Example 1.
Fig. 5 is a graph showing time-dependent change in yeast cells.
Fig. 6 is a chart showing the results of analysis of extracted total RNA purity with a bioanalyzer.
Fig. 7 is a photograph showing fluorescence detected for four blocks forming a row at the edge in the direction of the short side of the DNA array.
Fig. 8 is an illustration showing the results of clustering, based on the fluorescence pattern on the DNA array.
Fig. 9 is a schematic diagram showing the position of the SBc33P17 region in *Saccharomyces cerevisiae* genomic DNA.

### Explanation of Symbols

1: Substrate, 2: plasmid, 3: known gene, 4: block, 10, 20, 30: DNA arrays.

### Best Mode for Carrying Out the Invention

Preferred embodiments of the DNA array and gene expression analysis method of the invention will now be explained with reference to the accompanying drawings. In explaining the drawings, corresponding elements will be referred to by like reference numerals and will be explained only once.

### (DNA array)

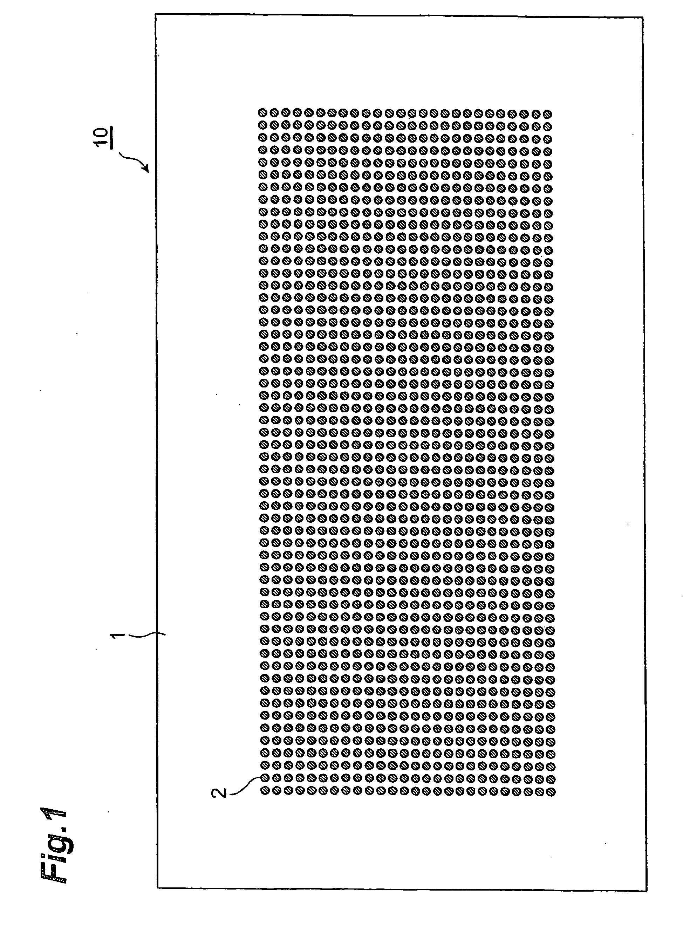

Fig. 1 is a plan view schematically showing a DNA array according to a first embodiment. The DNA array 10 of the first embodiment is composed of a substrate 1 and plasmids 2 binding at a plurality of spots on the substrate 1. In the DNA array 10, the plurality of spots binding the plasmids 2 are arranged in a lattice fashion on the substrate 1.

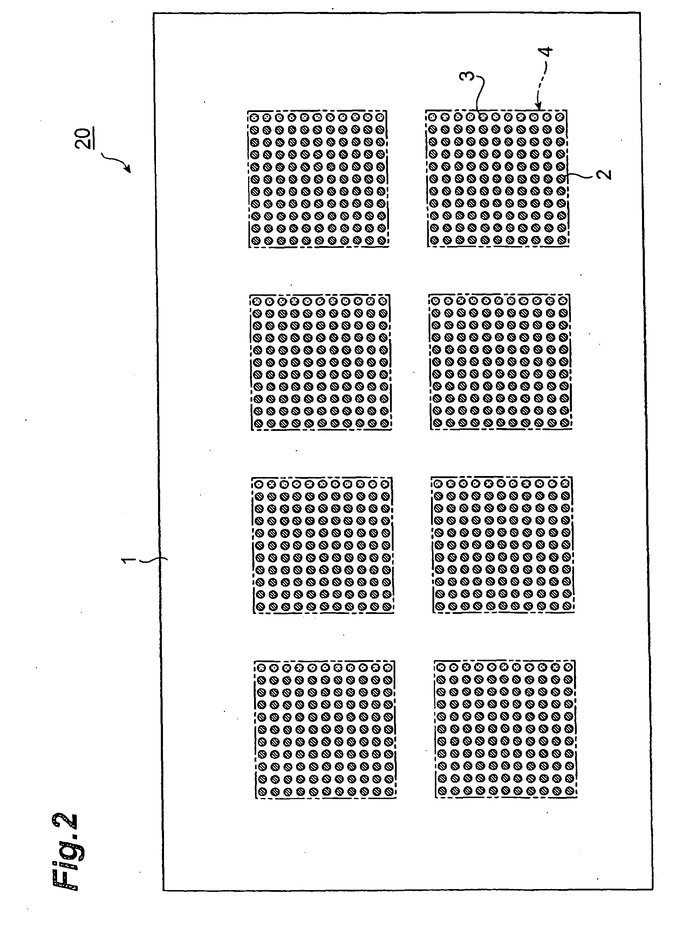

Fig. 2 is a plan view schematically showing a DNA array according to a second embodiment. The DNA array 20 of the second embodiment comprises a substrate 1, plasmids 2 binding to a plurality of spots on the substrate 1, and a brewer's yeast gene 3 of known function binding to another spot on the substrate 1. In the DNA array 20, a plurality of blocks 4 composed of the plurality of spots binding the plasmids 2 and the spot binding the known gene 3 are arranged on the substrate 1. The plurality of blocks 4 have equal shapes, and the binding spot of the known gene 3 is at the same location of each block 4. The blocks 4 are composed of spots arranged in a lattice fashion, and the blocks 4 are also arranged in a lattice fashion on the substrate 1.

The material of the substrate 1 of the DNA array 10 or 20 may be any one that can stably bind the plasmids 2, and for example, there may be mentioned glass and synthetic resins, as well as polycarbonate, plastic and the like. The form of the substrate 1 is preferably a sheet or film.

The plasmids 2 include genomic DNA fragments of brewer's yeast. The brewer's yeast is preferably bottom-fermenting yeast, and more preferably *Saccharomyces pastorianus Weihenstephan* 34/70 which is the most widely used yeast for beer brewing.

The sizes of the genomic DNA fragments in the plasmids 2 are preferably 1.5-3.0 kbp. If they are shorter than 1.5 kbp, the number of require genomic DNA fragments will tend to be increased when it is attempted to prepare a DNA array comprising the brewer's yeast genomic DNA in a comprehensive manner, and this will result in increased cost for preparing the DNA array. If they are longer than 3.0 kbp, on the other hand, there will tend to be more genes or exons in a single DNA fragment, making the process of gene expression analysis more complex.

The actual redundancy of the DNA array (the value that is the total of the lengths of the genomic DNA fragments on the DNA array divided by the genome size of the brewer's yeast used to prepare the DNA array) is preferably between 1.5 and 2.0. An actual redundancy of less than 1.5 will tend to reduce the size of the genomic region covered by the entirety of genomic DNA fragments on the DNA array, and if the actual redundancy is greater than 2.0 there will be larger regions of overlap between genomic DNA fragments on different spots, thus tending to complicate gene expression analysis.

Incidentally, brewer's yeast genomic DNA fragments which are the inserts of the plasmids 2 may be directly bonded to the DNA array instead of the plasmids 2. In this case as well, the sizes and redundancy of the genomic DNA fragments are preferably the same as when using the plasmids 2.

The known gene 3 on the DNA array 20 can be selected by the experimenter according to the purpose. The known gene 3 is preferably a gene derived from *Saccharomyces cerevisiae,* whose sequence data can be obtained from the Saccharomyces Genome DataBase (http://www.yeastgenome.org/).

The plasmids 2 on the DNA array 20 preferably differ depending on the block. The known gene 3 is preferably bonded at the same position of each block.

When a plurality of different known genes 3 are present on each block 4, at least one of them is preferably a gene that is constitutively expressed regardless of the strain or state of the brewer's yeast. Having such a gene on each block will be capable to easily evaluating the performance of the hybridization experiments at each block. One such gene that may be mentioned is the ACT1 gene derived from *Saccharomyces cerevisiae.*

If a plurality of spots binding plasmids or known genes are arranged in a lattice fashion as in the DNA array 10 or 20, the spot location can be represented by coordinates. Thus, it is possible to easily identify of spot locations and convenient for gene expression analysis. In the DNA array 20, preferably the same known gene is bound at the same location of each spot.

A DNA array according to the invention is prepared by binding plasmids, randomly selected from a random genomic library of brewer's yeast, onto a substrate surface.

The random genomic library of brewer's yeast may be prepared by the following procedures 1)-6). Each procedure may be carried out by a publicly known method, unless otherwise specified.
1) Genomic DNA is extracted and purified from brewer's yeast.
2) The extracted and purified genomic DNA is fragmented. The genomic DNA is preferably fragmented using a DNA fragmenting device in order to obtain random DNA fragments.
3) Genomic DNA fragments of a fixed size are recovered from the obtained genomic DNA fragments by electrophoresis.
4) The obtained genomic DNA fragments are blunt ended and ligated into an appropriate plasmid vector.
5) The obtained recombinant vector is introduced into appropriate host cells.
6) The host cells are cultured on appropriate medium and transformed cell are selected.

Binding of the plasmid to the substrate surface can be accomplished by a method in which, for example, a fixing solution dissolving the genomic DNA fragment-containing plasmids is spotted on a surface-treated (for example, polylysine-treated) substrate with a spotter, and the plasmids are thus immobilized on the substrate surface. Using an alkaline fixing solution as the fixing solution will separate each of the double-stranded plasmids into two single-stranded plasmids in the fixing solution. When a non-alkaline fixing solution is used, the double strands may be converted to single strands by heat treatment or the like.

### (Gene expression analysis method)

The gene expression analysis method of the invention comprises the following steps.
(a) A step of extracting total RNA from two kinds of brewer's yeasts of two different strains or states.
(b) A step of obtaining cDNA or cRNA labeled with a first fluorescent dye from the total RNA of one kind, and obtaining cDNA or cRNA labeled with a second fluorescent dye from the total RNA of the other kind.
(c) A step of competitively hybridizing the two different labeled cDNAs or two different labeled cRNAs on a DNA array comprising plasmids containing genomic DNA fragments of a brewer's yeast, either identical to or different from the brewer's yeast, bound onto a plurality of spots on the substrate.
(d) A step of measuring the fluorescence emitted by the first and second fluorescent dye at each spot.
(e) A step of comparing the intensity of fluorescence emitted by the first fluorescent dye with the intensity of fluorescence emitted by the second fluorescent dye.
(f) A step of analyzing the sequences of the genomic DNA fragments in the plasmids at spots where differences in fluorescent intensity are observed.

The "different strains or states" of brewer's yeast in step (a) means that the strain of brewer's yeast itself is different, or that the brewer's yeast growth conditions (temperature, growth time, medium composition, degree of aeration through medium, etc.) and the fermentation conditions (fermentation temperature, fermentation time, etc.) are different.

The extraction of total RNA in step (a) may be carried out by, for example, the hot phenol method (method of extracting RNA while lysing the yeast with heated phenol and sodium dodecylsulfate (SDS)), but it is preferably carried out to obtain high purity RNA of low solubility by freezing the yeast with liquid nitrogen, and then crushing them with a crusher and extracting the RNA with an RNA extraction reagent. The crusher may be a Cryopress (product of Microtec Co., Ltd.) and the RNA extraction reagent may be Isogen^{™} (product of Nippon Gene Co., Ltd.).

The labeled cDNA or labeled cRNA in step (b) may be prepared, for example in the case of labeled cDNA, by annealing oligo dT primer to total RNA extracted from the brewer's yeast and synthesizing cDNA using reverse transcriptase in the presence of fluorescent dye-bonded dNTP, and removing off the unreacted substrate with a spin column. An oligo dT column may be used for isolation and purification of poly A⁺ RNA from the total RNA before the reverse transcription reaction.

Moreover, labeled cRNA may be prepared by synthesizing unlabeled cDNA from total RNA or poly A⁺ RNA by reverse transcription reaction using oligo dT primer including the T7 promoter sequence, and then synthesizing cRNA by reacting T7 RNA polymerase reacted with the obtained cDNA in the presence of fluorescent dye-bonded NTP, and removing the unreacted substrate with a spin column.

The fluorescent dye used for labeling of the cDNA or cRNA is the one capable of distinguishing yeast strains or states based on color (fluorescent wavelength), and a cyanine dye is preferred. As examples of cyanine dyes with different colors there may be mentioned Cy3 (green dye) and Cy5 (red dye).

The DNA array used in step (c) preferably also comprises another spot binding a known gene.
As a suitable type of DNA array, there may be used one wherein blocks consisting of a plurality of spots binding plasmids including brewer's yeast genomic DNA fragments and a spot binding the known gene, are arranged on a substrate. Particularly, the one having blocks with equal shapes where the spots binding the known gene are located at the same position of each block is preferred.

The competitive hybridization in step (c) is carried out competitively on a single DNA array. For hybridization of cDNA, the double-strands are converted to single strands by heat treatment prior to the hybridization. The hybridization may be carried out, for example, by the method described in "DNA Arrays and State-of-the-Art PCR Methods" (Shujunsha Publishing).

Instead of the competitive hybridization as in step (c) above, hybridization may be carried out on separate DNA arrays for different yeast strains or states (providing that the same plasmids or known gene are bound at the same positions of each DNA array). In this case, the fluorescent dye used for labeling the cDNA or cRNA may be different or the same for each strain or state of yeast.

Measurement of the fluorescence intensity in step (d) may be accomplished by acquiring image data with a CCD camera or scanner and digitizing it with software.

The comparison of the fluorescence intensities in step (e) can be accomplished by calculating the ratio between the fluorescence intensities of the first and second fluorescent dye, measured in step (d).

For sequence analysis of the DNA fragments in step (f), the nucleotide sequences of the genomic DNA fragments are determined, and then the genes in the DNA fragments are identified and the functions of the genes predicted. Specifically, where differences in measured fluorescence intensities are observed, the nucleotide sequences of those genomic DNA fragments are determined, and these are associated with the strain and state of yeast, the genes are identified by database research (homology search or the like), and the function of the gene in each genomic DNA fragment is predicted. Detection of genomic DNA fragments where differences in measured fluorescence intensities are observed is preferably accomplished by repeating steps (a)-(e) several times and clustering the results based on similarities in the expression ratio pattern. The sequence analysis may be carried out using a plasmid in the library corresponding to the genomic DNA fragment being analyzed.

When a DNA array 20 is used in step (c), it is possible to identify a gene present in a DNA fragment and estimate its function by analysing the sequence of a genomic DNA fragment at a spot exhibiting a pattern of fluorescence intensity (proportion of fluorescence intensity between the first and second fluorescent dye) similar to or in synchrony with that of the spot binding the known gene. In this case, the nucleotide sequences of the genes are determined, they are associated with the function of the known gene, and a database research (homology search or the like) is conducted to predict the function of the gene in each genomic DNA fragment. Detection of genomic DNA fragments exhibiting a pattern of fluorescence intensity similar to or in synchrony with the known gene is preferably accomplished by repeating steps (a)-(e) several times and clustering the results based on similarities in the expression ratio pattern.

The gene expression analysis method of the invention preferably comprises, before reaching step (f), a step of identifying the gene region for ribosomal RNA among the genomic DNA fragments on the DNA array. If the gene region of ribosomal RNA is identified, it will be possible to accomplish highly precise analysis by excluding such regions from the sequence analysis.

The gene region for ribosomal RNA may be identified by the following procedure with steps 1)-3).
1) A portion of the 18S ribosomal RNA gene and a portion of the 28S ribosomal RNA gene are used as primers with brewer's yeast genomic DNA used as template, for PCR amplification of DNA fragments complementary to the 18S ribosomal RNA gene region and 28S ribosomal RNA gene region in the brewer's yeast genomic DNA.
2) The obtained DNA fragments are labeled with a fluorescent dye, radioactive isotope or the like.
3) The labeled DNA fragments are hybridized with plasmids on the DNA array.
4) The label is detected.

### Examples

Examples of the invention will now be explained. However, the invention is in no way limited to these examples.

### (Example 1: Preparation of DNA array)

### Preparation of random genomic library:

A random genomic library for brewer's yeast was prepared using *Saccharomyces pastorianus Weihenstephan* 34/70 as the brewer's yeast.

Extraction and purification of genomic DNA from the brewer's yeast was carried out by the method described in, for example, Methods in Yeast Genetics, A Cold Spring Harbor Laboratory Course Manual (Sadakazu Oya, ed., Maruzen Publishing, p.113-119)

The random genomic library was prepared in the following manner. 1) The genomic DNA was fragmented using a DNA fragmenting device (HydroShear). 2) The obtained genomic DNA fragments were subjected to electrophoresis on agarose gel, and an approximately 2.5 kbp DNA fragment was cut out. 3) The genomic DNA fragment ends were blunt ended and ligated into pUC19 vector. 4) The obtained recombinant vector was introduced into *E. coli* and glycerol stock was prepared from the resulting colonies. 5) The *E. coli* cells were transferred from the glycerol stock to LB medium (10 g/L bactotryptone, 5 g/L yeast extract, 10 g/L sodium chloride, pH 7.0) (liquid medium), and approximately 20,000 plasmid clones were extracted. Since each plasmid clone contained an approximately 2.5 kbp genomic DNA fragment, the entirety of the extracted plasmid clones included a genomic DNA fragment of approximately 50Mbp (2.5 kbp × 20,000 clones), and therefore it was estimated to have about 2.0 redundancy of the bottom-fermenting yeast genome size of 24-26 Mbp.

### Evaluation of random genomic library:

The random genomic library was evaluated in the following manner. 1) Plasmid DNA was digested with restriction enzyme BamHI and then subjected to agarose gel electrophoresis. 2) Base sequence analysis was then performed with vector primers, using as template plasmid DNA dispensed in eight 96-well plates. As a result, approximately 90% of the clones were confirmed to have genomic DNA inserts. That is, the actual redundancy of the prepared DNA array was estimated to be about 1.8 (2.0 × 0.9).

### Preparation of DNA array:

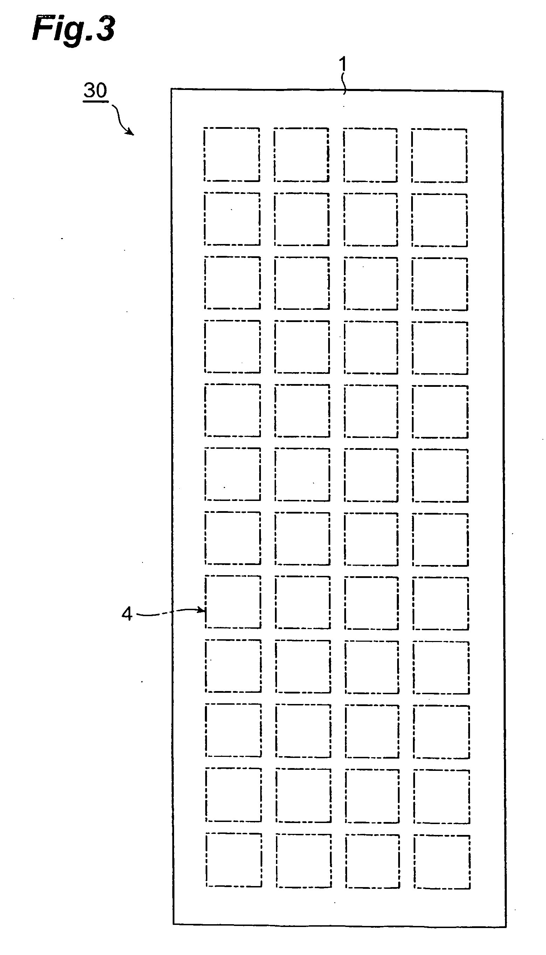

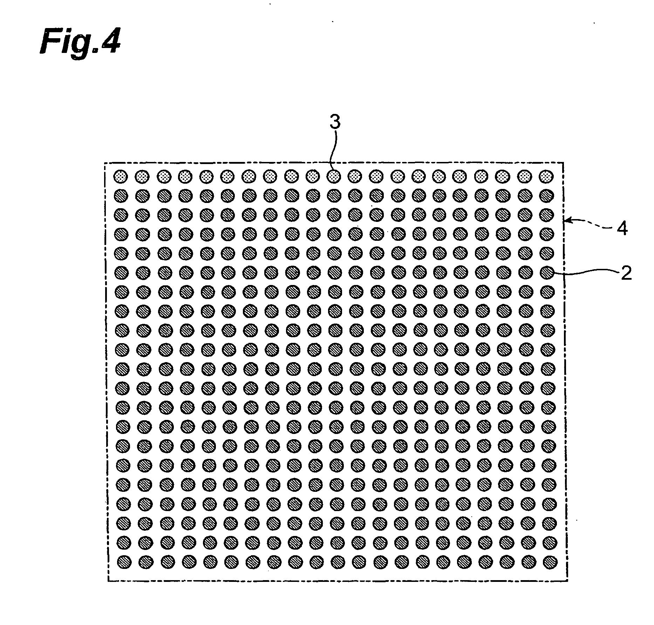

Fig. 3 is a plan view schematically showing the DNA array prepared in Example 1. As shown in Fig. 3, the DNA array 30 prepared in Example 1 was composed of blocks 4 with 12 × 4 rows (total 48) arranged on the substrate 1. Fig. 4 is a plan view schematically showing a magnified view of one block 4 of the DNA array 30. The block 4 is composed of 21 × 21 spots binding plasmids 2 or a known gene 3. The 21 spots binding the known gene 3 form a row at the edge of each block 4 in the direction of the short side of the DNA array 30. In Fig. 4, the blank is shown as the known gene 3 for convenience. Known genes 3 bound to different spots may be different kind of genes with each other.

The preparation of DNA array 30 was accomplished by immobilizing plasmids 2 on the spots on rows 2-21 of each block 4, and the known gene 3 on the first row spots of each block 4. A glass panel was used as the substrate 1, and the immobilization was accomplished by spotting the plasmids 2 or known gene 3 dissolved in an alkaline fixing solution on the glass panel after surface treatment (polylysine treatment).

The known gene 3 was one derived from *Saccharomyces cerevisiae*, and the DNA amplified by PCR was fixed. The names, functions and DNA amplification primers for the known genes fixed on the DNA array are listed as 1)-10) below.
1) Gene name: ACT1, Function: actin production, Primers: (5'-TCGGT AGACC AAGAC ACCAA-3', 5'-CCTTA CGGAC ATCGA CATCA-3')
2) Gene name: ATF1, Function: acetic acid ester synthesis, Primers: (5'-GCCAC ATCCA GTGCA TGATT-3', 5'-TAGTT GTGAG CGGCA ATCTG-3')
3) Gene name: ATF2, Function: acetic acid ester synthesis, Primers: (5'-CGAAG AGGCC TAATT GGAGA-3', 5'-TCACC GTTGT CGTAC GATTC-3')
4) Gene name: Lg-ATF1, Function: acetic acid ester synthesis, Primers: (5'-GGTGT GATTC TCAAC GAGCA-3', 5'-AACGG AGTGA TGGTG CACTT-3')
5) Gene name: EHT1, Function: lipid metabolism, Primers: (5'-TACCA GAGGT TGTGC ACGTT-3', 5'-TCTGC AATTG CCTTG GTAGC-3')
6) Gene name: IAH1, Function: esterase activity, Primers: (5'-GATCA GTATG CTCTT GGAGC-3', 5'-GTTGT TGCCA AGCAT CACCA-3')
7) Gene name: LEU4, Function: isopropyl malate synthesis, Primers: (5'-ACGGT GGAAG CATTA ACAGG-3', 5'-GGATC CAATG GCAAG TATGG-3')
8) Gene name: OLE1, Function: fatty acid desaturation, Primers: (5'-CTCCG TTTTC TACTA CGCTG-3', 5'-GTTGG GTCGT ATTGG TACCA-3')
9) Gene name: SSU1, Function: sulfite transport, Primers: (5'-TGCTC TTACG AGGCA GTTTG-3', 5'-ATGGC ATGCA GCCAC GTTAA-3')
10) Gene name: Lg-FLO1 Function: aggregating gene, Primers: (5'-CAACA AAGCA AACCA AGGGG-3', 5'-TTACC ATACG ATTGC CAGCA-3')

Fixed on each block 4, at the spots forming the row at the edge in the direction of the short side of the DNA array 30, were 1: ACT1, 2: ATF1, 3: ATF2, 4: Lg-ATF1, 5: EHT1, 6: IAH1, 7: LEU4, 8: OLE1, 9-18: blank, 19: pUC19 (control), 20: SSU1, 21: Lg-FLO1. Specifically, there were fixed on the glass substrate surface of the DNA array 30, 528 (11 × 4 rows × 12 columns) known genes (including a control) and 20,160 (20 × 21 × 4 rows × 12 columns) of plasmids. The ACT1 gene is a gene constitutively expressed regardless of the strain or state of the brewer's yeast.

### Identification of ribosomal RNA gene regions:

In order to identify the ribosomal RNA gene regions overlapping on the genomic DNA fragments on the DNA array, DNA fragments having the sequences shown below were used as primers for the 18S ribosomal RNA gene and 28S ribosomal RNA gene, with bottom-fermenting yeast genomic DNA as template, for PCR amplification of complementary DNA fragments for the 18S ribosomal RNA gene region and 28S ribosomal RNA gene region among the genomic DNA of the bottom-fermenting yeast.
18S ribosomal RNA gene primers:
   (5'-CTGGT TGATY CTGCC AGT-3', 5'-CYGCA GGTTC ACCTA CRG-3')
28S ribosomal RNA gene primers:
   (5'-RCATC GATGA AGAAC GYWG-3', 5'-MRGGC TKAAT CTCAR YRGAT CG-3')

The obtained DNA fragments were labeled with Cy5 and used as probes in hybridization on the DNA array. The hybridization was carried out for 14 hours in a thermostatic bath at 62°C following the method described in, for example, "DNA Arrays and State-of-the-Art PCR Methods" (Shujunsha Publishing).

As a result, of the 20,160 genomic DNA fragments fixed on the substrate, 1129 (approximately 6%) of the genomic DNA fragments were judged to correspond to ribosomal RNA gene regions and were masked in the subsequent analysis.

### (Example 2: bottom-fermenting yeast gene expression analysis)

### Extraction of total RNA:

A fermentation test using bottom-fermenting yeast was conducted under the following conditions.
· Wort extract concentration: approximately 11 %
· Fermentation scale: 2.5 L
· Wort dissolved oxygen concentration: approximately 5-10 ppm
· Fermentation temperature: approximately 13°C
· Pitching Yeast: 20-24 g wet yeast cells
· Number of fermentations: 3

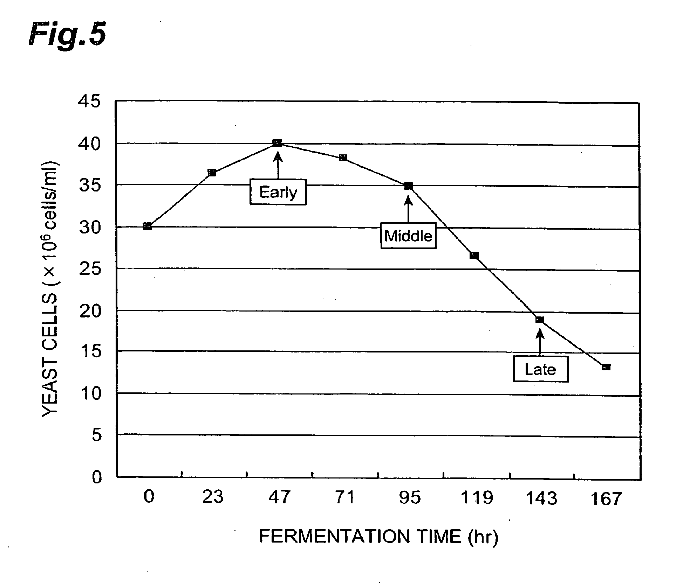

The fermenting wort was periodically sampled, and the time-dependent change in yeast cells (yeast proliferation) was examined. Yeast cells in fermenting wort were sampled at the early, middle and late stage of fermentation. Specifically, sampling was performed at 47, 95 and 143 hours from the start of fermentation. Fig. 5 is a graph showing the time-dependent change in yeast cells.

Total RNA was extracted in the following manner from yeast at each of the aforementioned fermentation points. 1) Yeasts (approximately 1 g) collected from the fermenting wort together with centrifugation tubes were frozen with liquid nitrogen. 2) The frozen cells were crushed with a Cryopress (Microtec Co., Ltd.) (crushing three times for 30 seconds). 3) The crushate was lysed with approximately 10 ml of Isogen^{™} (Nippon Gene Co., Ltd.) and the total RNA was purified according to the manufacturer's manual.

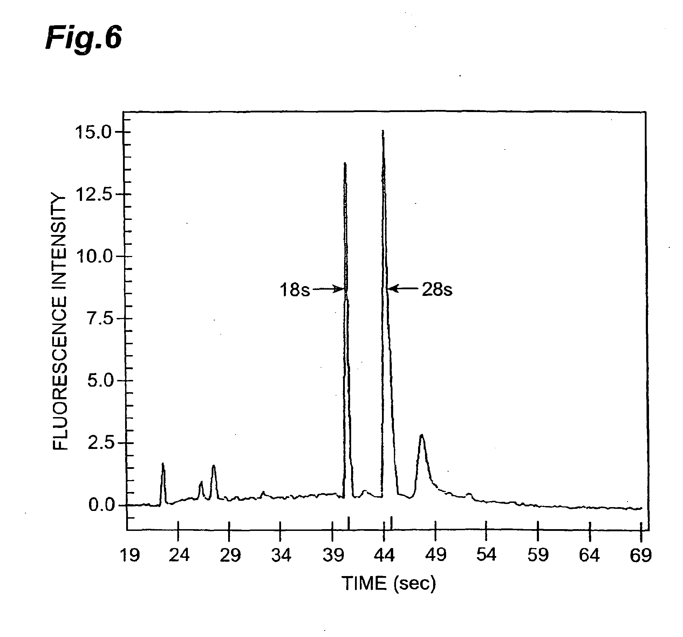

The concentration of total RNA extracted from the late-stage fermentation yeast was measured by spectrophotometry and the quality was confirmed using a bioanalyzer (Agilent 2100 Bioanalyzer). The concentration was 231.79 ng/µL. Fig. 6 is a chart showing the results of analysis of extracted total RNA purity with a bioanalyzer. As shown in Fig. 6, the proportion of 18S and 28S ribosomal RNA in the extracted total RNA was 1:1.6, and no 28S ribosomal RNA degradation product was detected. The results indicated that high purity total RNA had been extracted.

### Labeling with fluorescent dye:

After annealing oligo dT primer to the total RNA, reverse transcriptase reaction was conducted in the presence of aminoallyl-dUTP, to prepare cDNA containing aminoallyl-dUTP. Coupling reaction was carried out to label the cDNA with cyanine dye (Cy3 or Cy5). One of the two kinds of cDNAs hybridized to the plasmid and known gene on each DNA array was labeled with Cy3 (green dye), while the other was labeled with Cy5 (red dye).

### Hybridization:

For each DNA array, the two fluorescent dye labeled cDNAs were competitively hybridized to the plasmids and known gene on the DNA array. The hybridization was carried out for 14 hours in a thermostatic bath at 62°C following the method described in, for example, "DNA Arrays and State-of-the-Art PCR Methods" (Shujunsha Publishing).

### Measurement of fluorescence intensity:

Following hybridization, the DNA array was rinsed and the signal was read with a slide scanner (Agilent Scanner, Agilent). The image data for the obtained signal were converted to numerical data using the proprietary software FeatureExtraction (Agilent). The fluorescence value of the negative control was used as the background, which was subtracted from the fluorescence value for each spot. Normalization between the sample and control (pUC19) was accomplished by the global normalization method (normalization using all spots).

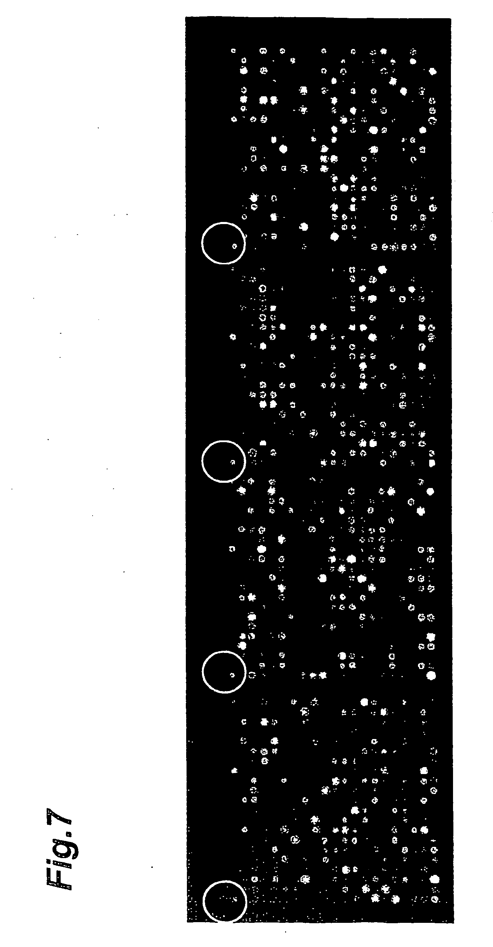

The performance of the hybridization experiment were judged from the fluorescence intensity at 48 spots (4 rows × 12 columns) for the ACT1 gene in the scanned image. Fig. 7 is a photograph showing fluorescence detected for four blocks forming a row at the edge in the direction of the short side of the DNA array. In Fig. 7, spots binding the known gene (including blanks) form a row at the edge on the short side of the DNA array, in each block. As shown in Fig. 7, the fluorescence intensity of the constitutively expressed ACT1 gene (indicated by white circles in the photograph) was approximately equivalent for each block, and therefore the results of the hybridization experiment were judged to be positive.

### Sequence analysis:

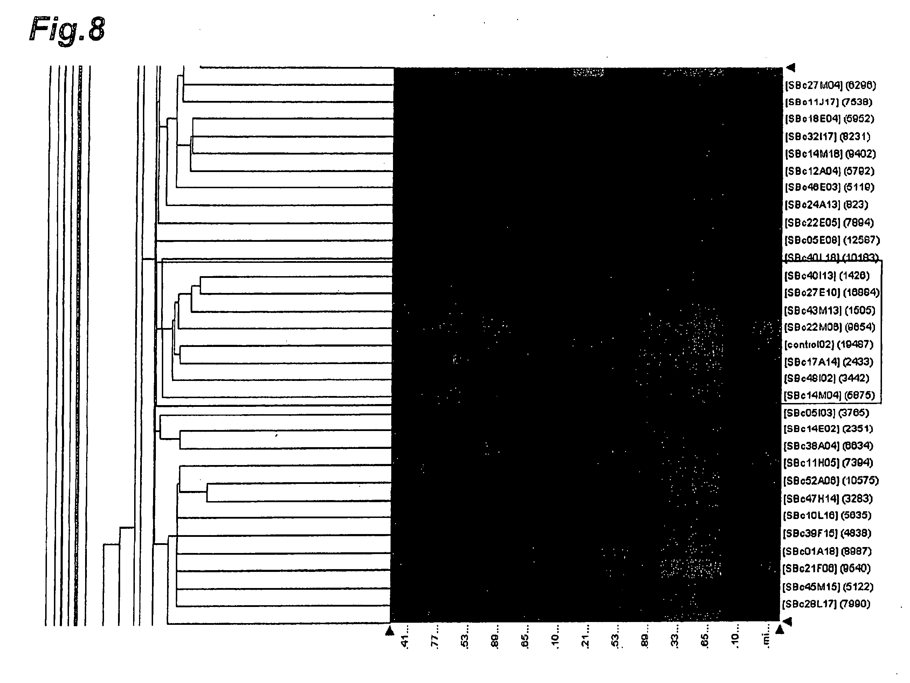

Clustering was performed based on fluorescence patterns on the DNA array obtained in three fermentation experiments. There were selected seven spots whose fluorescence patterns were in synchrony with that of the spot fixing the ATF1 gene coding for alcohol acetyltransferase as the known genes. For each of the spots, the sequences of the corresponding genomic DNA fragments in the random genomic library were analyzed and the functions of the genes were estimated from a database research using BLAST or the like. As a result, the ten genes listed in Table 1 were annotated from six genomic DNA fragments (SBc40I13, SBc48I02, SBc14M04, SBc43M13, SBc27E10 and SBc22M06), and of the genomic DNA fragments (SBc17A14) was a non-*Saccharomyces cerevisiae* type. Fig. 8 is an illustration showing the results of clustering, based on the fluorescence pattern on the DNA array. In Fig. 8, control 02 represents the ATF1 gene.

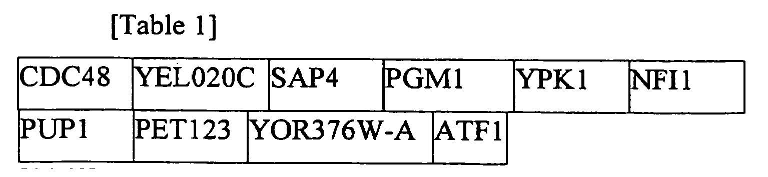

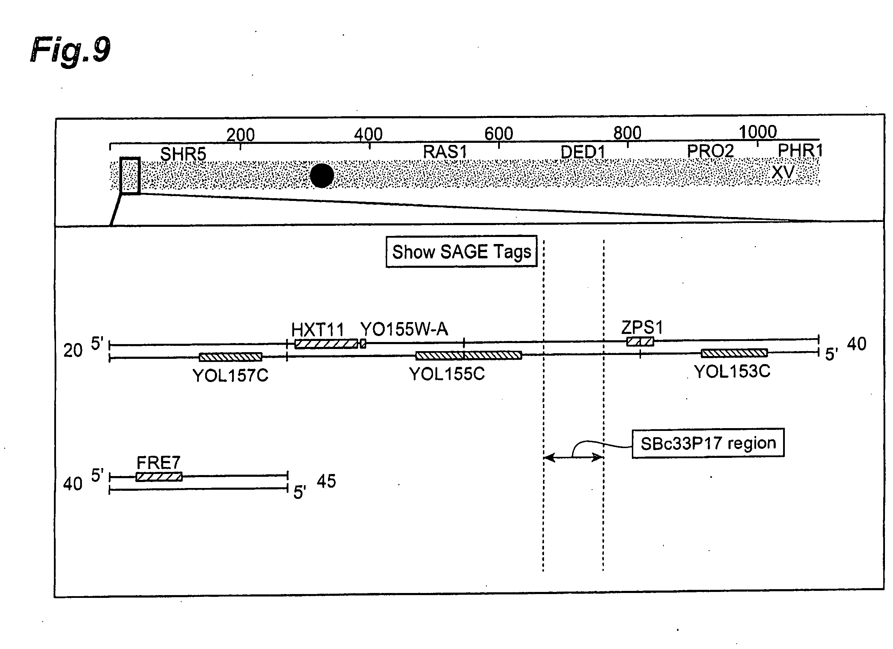

As a result of clustering, five spots were selected that had increasing gene expression during the fermentation process. Sequence analysis showed that the five genomic DNA fragments (SBc25J18, SBc35O20, SBc42N04, SBc32N16 and SBc33P17) corresponding to these spots were genomic DNA fragments equivalent to *Saccharomyces cerevisiae.* Fig. 9 is a schematic diagram showing the position of the SBc33P17 region in *Saccharomyces cerevisiae* genomic DNA. As shown in Fig. 9, SBc33P17 corresponds to a region of 32250-34492 bp on chromosome XV of *Saccharomyces cerevisiae*, but this region does not include an open reading frame and is therefore a non-coding region.

### [Industrial Applicability]

The present invention provides a gene expression analysis method that can be satisfactorily used for the purpose of controlling the brewer's yeast fermentation process without requiring previous acquisition of brewer's yeast genomic sequence data, as well as a DNA array used in the gene expression analysis method. The DNA array and gene expression analysis method of the invention can be utilized for production of alcoholic beverages that are prepared by fermentation of yeast.

## Claims

1. A DNA array comprising plasmids containing genomic DNA fragments of brewer's yeast bound onto a plurality of spots on a substrate.

2. A DNA array according to claim 1, which comprises another spot binding a brewer's yeast gene of known function.

3. A DNA array according to claim 2, wherein on the substrate there are arranged a plurality of blocks, each composed of a plurality of spots binding plasmids containing genomic DNA fragments of brewer's yeast, and a spot binding a brewer's yeast gene of known function.

4. A DNA array according to claim 3, wherein the blocks have equal shapes, and the spots binding the brewer's yeast gene of known function are situated at the same position of each block.

5. A DNA array according to any one of claims 1 to 4, wherein the brewer's yeast is bottom-fermenting yeast.

6. A gene expression analysis method for brewer's yeast, comprising
(a) a step of extracting total RNA from two kinds of brewer's yeasts of different species or states,
(b) a step of obtaining cDNA or cRNA labeled with a first fluorescent dye from the total RNA of one kind and obtaining cDNA or cRNA labeled with a second fluorescent dye from the total RNA of the other kind,
(c) a step of competitively hybridizing the two different labeled cDNAs or two different labeled cRNAs on a DNA array comprising plasmids containing genomic DNA fragments of brewer's yeast, either identical to or different from the brewer's yeast, bound onto a plurality of spots on a substrate,
(d) a step of measuring the fluorescence emitted by the first and second fluorescent dyes at each spot,
(e) a step of comparing the intensity of fluorescence emitted by the first fluorescent dye with the intensity of fluorescence emitted by the second fluorescent dye, and
(f) a step of analyzing the sequences of the genomic DNA fragments in the plasmids at spots where a difference in fluorescent intensity is observed.

7. A gene expression analysis method for brewer's yeast, comprising
(a) a step of extracting total RNA from two kinds of brewer's yeasts of different strains or states,
(b) a step of obtaining the following (b1) or (b2) from the total RNA:
(b1) first labeled cDNA labeled with a first fluorescent dye and second labeled cDNA labeled with a second fluorescent dye,
(b2) first labeled cRNA labeled with a first fluorescent dye and second labeled cRNA labeled with a second fluorescent dye,
(c) a step of preparing two DNA arrays binding plasmids containing genomic DNA fragments of brewer's yeast identical to or different from the brewer's yeast at a plurality of spots on a substrate where the two DNA arrays are identical and one is designated as the first DNA array while the other is designated as the second DNA array, and hybridizing them in a manner according to (c1) or (c2) below:
(c1) first labeled cDNA on the first DNA array and second labeled cDNA on the second DNA array,
(c2) first labeled cRNA on the first DNA array and second labeled cRNA on the second DNA array,
(d) a step of measuring the fluorescence emitted by the first fluorescent dye and second fluorescent dye at each corresponding spot of first DNA array and second DNA array,
(e) a step of comparing the intensity of fluorescence emitted by the first fluorescent dye with the intensity of fluorescence emitted by the second fluorescent dye, and
(f) a step of analyzing the sequences of the genomic DNA fragments in the plasmids at spots where a difference in fluorescent intensity is observed.

8. A gene expression analysis method according to claim 6 or 7, wherein the DNA array used in step (c) further comprises another spot binding a brewer's yeast gene of known function.

9. A gene expression analysis method according to claim 8, wherein in the DNA array used in step (c), a plurality of blocks consisting of a plurality of spots binding plasmids including brewer's yeast genomic DNA fragments and a spot binding the brewer's yeast gene of known function, are arranged on a substrate.

10. A gene expression analysis method according to claim 9, wherein in the DNA array used in step (c), the blocks have equal shapes, and the spots binding the brewer's yeast gene of known function are situated at the same position of each block.

11. A gene expression analysis method according to any one of claims 6 to 10, wherein the brewer's yeast in step (c) is bottom-fermenting yeast.

## Patentansprüche

1. DNA-Array, umfassend Plasmide, die genomische DNA-Fragmente von Brauhefe enthalten, die auf einer Vielzahl von Flecken auf einem Substrat gebunden sind.

2. DNA-Array nach Anspruch 1, das einen weiteren Fleck umfasst, der ein Brauhefegen von bekannter Funktion bindet.

3. DNA-Array nach Anspruch 2, wobei auf dem Substrat eine Vielzahl von Feldern angeordnet sind, wobei jedes zusammengesetzt ist aus einer Vielzahl von Flecken, die Plasmide binden, die genomische DNA-Fragmente von Brauhefe enthalten, und einem Fleck, der ein Brauhefegen von bekannter Funktion bindet.

4. DNA-Array nach Anspruch 3, wobei die Felder die gleiche Form haben und die Flecken, die das Brauhefegen von bekannter Funktion binden, an derselben Stelle in jedem Feld positioniert sind.

5. DNA-Array nach einem der Ansprüche 1 bis 4, wobei die Brauhefe untergärige Hefe ist.

6. Verfahren zur Genexpressionsanalyse von Brauhefe, umfassend:
(a) einen Schritt des Extrahierens von Gesamt-RNA von zwei Arten von Brauhefe unterschiedlicher Spezies oder Zustände,
(b) einen Schritt des Erhaltens von cDNA oder cRNA, die mit einem ersten Fluoreszenzfarbstoff markiert ist, aus Gesamt-RNA einer Art, und Erhalten von cDNA oder cRNA, die mit einem zweiten Fluoreszenzfarbstoff markiert ist, aus Gesamt-RNA der anderen Art,
(c) einen Schritt des kompetitiven Hybridisierens von zwei verschiedenen markierten cDNAs oder zwei verschiedenen markierten cRNAs auf einem DNA-Array, umfassend Plasmide, die genomische DNA-Fragmente von Brauhefe enthalten, die entweder identisch oder unterschiedlich zu der Brauhefe ist, die auf einer Vielzahl von Flecken auf dem Substrat gebunden sind,
(d) einen Schritt des Messens der emittierten Fluoreszenz von dem ersten und dem zweiten Fluoreszenzfarbstoff an jedem Fleck;
(e) einen Schritt des Vergleichens der Intensität der emittierten Fluoreszenz des ersten Fluoreszenzfarbstoffs mit der Intensität der emittierten Fluoreszenz des zweiten Fluoreszenzfarbstoffs, und
(f) einen Schritt des Analysierens der Sequenz der genomischen DNA-Fragmente in den Plasmiden an Flecken, an denen ein Unterschied in der Fluoreszenzintensität beobachtet wird

7. Verfahren zur Genexpressionsanalyse von Brauhefe, umfassend:
(a) einen Schritt des Extrahierens von Gesamt-RNA von zwei Arten von Brauhefe unterschiedlicher Spezies oder Zustände,
(b) einen Schritt des Erhaltens der folgenden (b1) oder (b2) aus Gesamt-RNA:
(b1) eine erste markierte cDNA, die mit einem ersten Fluoreszenzfarbstoff markiert ist, und eine zweite markierte cDNA, die mit einem zweiten Fluoreszenzfarbstoff markiert ist,
(b2) eine erste markierte cRNA, die mit einem ersten Fluoreszenzfarbstoff markiert ist, und eine zweite markierte cRNA, die mit einem zweiten Fluoreszenzfarbstoff markiert ist,
(c) einen Schritt des Herstellens von zwei DNA-Arrays, die Plasmide, die genomische DNA-Fragmente von Brauhefe enthalten, die identisch oder unterschiedlich zu der Brauhefe ist, an einer Vielzahl von Flecken auf einem Substrat binden, wobei die DNA-Arrays identisch sind und eines als das erste DNA-Array bezeichnet ist und das andere als das zweite DNA-Array bezeichnet ist, und Hybridisieren der beiden in einer Weise gemäß (c1) oder (c2) unten:
(c1) die erste markierte cDNA auf dem ersten DNA-Array und die zweite markierte cDNA auf dem zweiten DNA-Array:
(c2) die erste markierte cRNA auf dem ersten DNA-Array und die zweite markierte cRNA auf dem zweiten DNA-Array,
(d) einen Schritt des Messens der emittierten Fluoreszenz von dem ersten Fluoreszenzfarbstoff und dem zweiten Fluoreszenzfarbstoff an jedem entsprechenden Fleck des ersten DNA-Arrays und des zweiten DNA-Arrays,
(e) einen Schritt des Vergleichens der Intensität der emittierten Fluoreszenz des ersten Fluoreszenzfarbstoffs mit der Intensität der emittierten Fluoreszenz des zweiten Fluoreszenzfarbstoffs, und
(f) einen Schritt des Analysierens der Sequenz der genomischen DNA-Fragmente in den Plasmiden an den Flecken, an denen ein Unterschied in der Fluoreszenzintensität beobachtet wird.

8. Verfahren zur Genexpressionsanalyse nach Anspruch 6 oder 7, wobei das DNA-Array, das in Schritt (c) verwendet wird, weiterhin einen Fleck umfasst, der ein Brauhefegen von bekannter Funktion bindet.

9. Verfahren zur Genexpressionsanalyse nach Anspruch 8, wobei in dem DNA-Array, das in Schritt (c) verwendet wird, eine Vielzahl von Feldern, die aus einer Vielzahl von Flecken, die Plasmide binden, die genomische DNA-Fragmente von Brauhefe umfassen, und einem Fleck, der das Brauhefegen bekannter Funktion bindet, bestehen, auf einem Substrat angeordnet sind.

10. Verfahren zur Genexpressionsanalyse nach Anspruch 9, wobei in dem DNA-Array, das in Schritt (c) verwendet wird, die Felder die gleiche Form haben und die Flecken, die das Brauhefegen von bekannter Funktion binden, an derselben Stelle in jedem Feld positioniert sind.

11. Verfahren zur Genexpressionsanalyse nach einem der Ansprüche 6 bis 10, wobei die Brauhefe aus Schritt (c) untergärige Hefe ist.

## Revendications

1. Puce à ADN comprenant des plasmides contenant des fragments d'ADN génomique de levure de bière liés sur une pluralité de points sur un substrat.

2. Puce à ADN selon la revendication 1, qui comprend un autre point se liant à un gène de levure de bière ayant une fonction connue.

3. Puce à ADN selon la revendication 2, dans laquelle est agencée sur le substrat une pluralité de blocs, composés chacun d'une pluralité de points se liant à des plasmides contenant des fragments d'ADN génomique de levure de bière, et un point se liant à un gène de levure de bière ayant une fonction connue.

4. Puce à ADN selon la revendication 3, dans laquelle les blocs ont des formes identiques, et les points se liant au gène de levure de bière ayant une fonction connue sont situés sur la même position de chaque bloc.

5. Puce à ADN selon l'une quelconque des revendications 1 à 4, dans laquelle la levure de bière est une levure de fermentation basse.

6. Procédé d'analyse de l'expression d'un gène pour la levure de bière, comprenant
(a) une étape d'extraction de l'ARN total de deux types de levures de bière d'espèces ou d'états différents,
(b) une étape d'obtention d'ADNc ou d'ARNc marqué par un premier colorant fluorescent, à partir de l'ARN total d'un premier type, et d'obtention d'ADNc ou d'ARNc marqué par un deuxième colorant fluorescent, à partir de l'ARN total de l'autre type,
(c) une étape d'hybridation de manière compétitive des deux ADNc marqués différents ou des deux ARNc marqués différents, sur une puce à ADN comprenant des plasmides contenant des fragments d'ADN génomique d'une levure de bière, soit identique à, soit différente de, la levure de bière, liés sur une pluralité de points sur un substrat,
(d) une étape de mesure de la fluorescence émise par les premier et deuxième colorants fluorescents au niveau de chaque point,
(e) une étape de comparaison de l'intensité de fluorescence émise par le premier colorant fluorescent avec l'intensité de fluorescence émise par le deuxième colorant fluorescent, et
(f) une étape d'analyse des séquences des fragments d'ADN génomique dans les plasmides au niveau des points où est observée une différence de l'intensité de fluorescence.

7. Procédé d'analyse de l'expression d'un gène pour la levure de bière, comprenant
(a) une étape d'extraction de l'ARN total de deux types de levures de bière d'espèces ou d'états différents,
(b) une étape d'obtention de (b1) ou (b2) suivant, à partir de l'ARN total :
(b1) un premier ADNc marqué, marqué par un premier colorant fluorescent, et un deuxième ADNc marqué, marqué par un deuxième colorant fluorescent,
(b2) un premier ARNc marqué, marqué par un premier colorant fluorescent, et un deuxième ARNc marqué, marqué par un deuxième colorant fluorescent,
(c) une étape de préparation de deux puces à ADN se liant à des plasmides contenant des fragments d'ADN génomique d'une levure de bière identique à, ou différente de, la levure de bière au niveau d'une pluralité de points sur un substrat, où les deux puces à ADN sont identiques et l'une est conçue comme la première puce à ADN tandis que l'autre est conçue comme la deuxième puce à ADN, et d'hybridation d'une manière conforme à (c1) ou (c2) ci-dessous :
(c1) le premier ADNc marqué sur la première puce à ADN et le deuxième ADNc marqué sur la deuxième puce à ADN,
(c2) le premier ARNc marqué sur la première puce à ADN et le deuxième ARNc marqué sur la deuxième puce à ADN,
(d) une étape de mesure de la fluorescence émise par le premier colorant fluorescent et le deuxième colorant fluorescent en chaque point correspondant de la première puce à ADN et de la deuxième puce à ADN,
(e) une étape de comparaison de l'intensité de fluorescence émise par le premier colorant fluorescent avec l'intensité de fluorescence émise par le deuxième colorant fluorescent, et
(f) une étape d'analyse des séquences des fragments d'ADN génomique dans les plasmides au niveau des points où est observée une différence de l'intensité de fluorescence.

8. Procédé d'analyse de l'expression d'un gène selon la revendication 6 ou 7, dans lequel la puce à ADN utilisée dans l'étape (c) comprend en outre un autre point se liant à un gène de levure de bière ayant une fonction connue.

9. Procédé d'analyse de l'expression d'un gène selon la revendication 8, dans lequel, dans la puce à ADN utilisée dans l'étape (c), une pluralité de blocs constitués d'une pluralité de points se liant à des plasmides contenant des fragments d'ADN génomique de levure de bière, et un point se liant à un gène de levure de bière ayant une fonction connue, sont agencés sur un substrat.

10. Procédé d'analyse de l'expression d'un gène selon la revendication 9, dans lequel, dans la puce à ADN utilisée dans l'étape (c), les blocs ont des formes identiques, et les points se liant au gène de levure de bière ayant une fonction connue sont situés sur la même position de chaque bloc.

11. Procédé d'analyse de l'expression d'un gène selon l'une quelconque des revendications 6 à 10, dans lequel la levure de bière dans l'étape (c) est une levure de fermentation basse.


### SEQUENCE LISTING

<110> Sapporo Breweries Ltd.
<120> DNA ARRAY AND METHOD FOR ANALYSIS OF GENE EXPRESSION OF BREWING YEAST
<130> EP53940HV163pau
<140> not yet assigned
   <141> 2006-02-27
<150> PCT/JP2006/303645
   <151> 2006-02-27
<150> 2005-059717
   <151> 2005-03-03
<160> 24
<170> PatentIn version 3.1
<210> 1
   <211> 20
   <212> DNA
   <213> Artificial
<220>
   <223> primer for ACT1
<400> 1
   tcggtagacc aagacaccaa 20
<210> 2
   <211> 20
   <212> DNA
   <213> Artificial
<220>
   <223> primer for ACT1
<400> 2
   ccttacggac atcgacatca 20
<210> 3
   <211> 20
   <212> DNA
   <213> Artificial
<220>
   <223> primer for ATF1
<400> 3
   gccacatcca gtgcatgatt 20
<210> 4
   <211> 20
   <212> DNA
   <213> Artificial
<220>
   <223> primer for ATF1
<400> 4
   tagttgtgag cggcaatctg 20
<210> 5
   <211> 20
   <212> DNA
   <213> Artificial
<220>
   <223> primer for ATF2
<400> 5
   cgaagaggcc taattggaga 20
<210> 6
   <211> 20
   <212> DNA
   <213> Artificial
<220>
   <223> primer for ATF2
<400> 6
   tcaccgttgt cgtacgattc 20
<210> 7
   <211> 20
   <212> DNA
   <213> Artificial
<220>
   <223> primer for Lg-ATF1
<400> 7
   ggtgtgattc tcaacgagca 20
<210> 8
   <211> 20
   <212> DNA
   <213> Artificial
<220>
   <223> primer for Lg-ATF1
<400> 8
   aacggagtga tggtgcactt 20
<210> 9
   <211> 20
   <212> DNA
   <213> Artificial
<220>
   <223> primer for EHT1
<400> 9
   taccagaggt tgtgcacgtt 20
<210> 10
   <211> 20
   <212> DNA
   <213> Artificial
<220>
   <223> primer for EHT1
<400> 10
   tctgcaattg ccttggtagc 20
<210> 11
   <211> 20
   <212> DNA
   <213> Artificial
<220>
   <223> primer for IAH1
<400> 11
   gatcagtatg ctcttggagc 20
<210> 12
   <211> 20
   <212> DNA
   <213> Artificial
<220>
   <223> primer for IAH1
<400> 12
   gttgttgcca agcatcacca 20
<210> 13
   <211> 20
   <212> DNA
   <213> Artificial
<220>
   <223> primer for LEU4
<400> 13
   acggtggaag cattaacagg 20
<210> 14
   <211> 20
   <212> DNA
   <213> Artificial
<220>
   <223> primer for LEU4
<400> 14
   ggatccaatg gcaagtatgg 20
<210> 15
   <211> 20
   <212> DNA
   <213> Artificial
<220>
   <223> primer for OLE1
<400> 15
   ctccgttttc tactacgctg 20
<210> 16
   <211> 20
   <212> DNA
   <213> Artificial
<220>
   <223> primer for OLE1
<400> 16
   gttgggtcgt attggtacca 20
<210> 17
   <211> 20
   <212> DNA
   <213> Artificial
<220>
   <223> primer for SSU1
<400> 17
   tgctcttacg aggcagtttg 20
<210> 18
   <211> 20
   <212> DNA
   <213> Artificial
<220>
   <223> primer for SSU1
<400> 18
   atggcatgca gccacgttaa 20
<210> 19
   <211> 20
   <212> DNA
   <213> Artificial
<220>
   <223> primer for Lg-FLO1
<400> 19
   caacaaagca aaccaagggg 20
<210> 20
   <211> 20
   <212> DNA
   <213> Artificial
<220>
   <223> primer for Lg-FLO1
<400> 20
   ttaccatacg attgccagca 20
<210> 21
   <211> 18
   <212> DNA
   <213> Artificial
<220>
   <223> primer for 185 rRNA
<220>
   <221> misc_feature
   <222> (10)..(10)
   <223> y = t/u or c
<400> 21
   ctggttgaty ctgccagt 18
<210> 22
   <211> 18
   <212> DNA
   <213> Artificial
<220>
   <223> primer for 18S rRNA
<220>
   <221> misc_feature
   <222> (2)..(2)
   <223> y = t/u or c
<220>
   <221> misc_feature
   <222> (10)..(10)
   <223> r = g or a
<400> 22
   cygcaggttc acctacrg 18
<210> 23
   <211> 19
   <212> DNA
   <213> Artificial
<220>
   <223> primer for 28s rRNA
<220>
   <221> misc_feature
   <222> (1)..(1)
   <223> r = g or a
<220>
   <221> misc_feature
   <222> (17)..(17)
   <223> y = t/u or c
<220>
   <221> misc_feature
   <222> (18)..(18)
   <223> w = a or t/u
<400> 23
   rcatcgatga agaacgywg 19
<210> 24
   <211> 22
   <212> DNA
   <213> Artificial
<220>
   <223> primer for 285 rRNA
<220>
   <221> misc_feature
   <222> (1)..(1)
   <223> m = a or c
<220>
   <221> misc_feature
   <222> (2)..(2)
   <223> r = g or a
<220>
   <221> misc_feature
   <222> (7)..(7)
   <223> k = g or t/u
<220>
   <221> misc_feature
   <222> (15)..(15)
   <223> r = g or a
<220>
   <221> misc_feature
   <222> (16)..(16)
   <223> y = t/u or c
<220>
   <221> misc_feature
   <222> (17)..(17)
   <223> r = g or a
<400> 24
   mrggctkaat ctcaryrgat cg 22

### SEQUENCE LISTING

<110> Sapporo Breweries Ltd.
<120> DNA ARRAY AND METHOD FOR ANALYSIS OF GENE EXPRESSION OF BREWING YEAST
<130> EP53940HV163pau
<140> not yet assigned
   <141> 2006-02-27
<150> PCT/JP2006/303645
   <151> 2006-02-27
<150> 2005-059717
   <151> 2005-03-03
<160> 24
<170> PatentIn version 3.1
<210> 1
   <211> 20
   <212> DNA
   <213> Artificial
<220>
   <223> primer for ACT1
<400> 1
   tcggtagacc aagacaccaa 20
<210> 2
   <211> 20
   <212> DNA
   <213> Artificial
<220>
   <223> primer for ACT1
<400> 2
   ccttacggac atcgacatca 20
<210> 3
   <211> 20
   <212> DNA
   <213> Artificial
<220>
   <223> primer for ATF1
<400> 3
   gccacatcca gtgcatgatt 20
<210> 4
   <211> 20
   <212> DNA
   <213> Artificial
<220>
   <223> primer for ATF1
<400> 4
   tagttgtgag cggcaatctg 20
<210> 5
   <211> 20
   <212> DNA
   <213> Artificial
<220>
   <223> primer for ATF2
<400> 5
   cgaagaggcc taattggaga 20
<210> 6
   <211> 20
   <212> DNA
   <213> Artificial
<220>
   <223> primer for ATF2
<400> 6
   tcaccgttgt cgtacgattc 20
<210> 7
   <211> 20
   <212> DNA
   <213> Artificial
<220>
   <223> primer for Lg-ATF1
<400> 7
   ggtgtgattc tcaacgagca 20
<210> 8
   <211> 20
   <212> DNA
   <213> Artificial
<220>
   <223> primer for Lg-ATF1
<400> 8
   aacggagtga tggtgcactt 20
<210> 9
   <211> 20
   <212> DNA
   <213> Artificial
<220>
   <223> primer for EHT1
<400> 9
   taccagaggt tgtgcacgtt 20
<210> 10
   <211> 20
   <212> DNA
   <213> Artificial
<220>
   <223> primer for EHT1
<400> 10
   tctgcaattg ccttggtagc 20
<210> 11
   <211> 20
   <212> DNA
   <213> Artificial
<220>
   <223> primer for IAH1
<400> 11
   gatcagtatg ctcttggagc 20
<210> 12
   <211> 20
   <212> DNA
   <213> Artificial
<220>
   <223> primer for IAH1
<400> 12
   gttgttgcca agcatcacca 20
<210> 13
   <211> 20
   <212> DNA
   <213> Artificial
<220>
   <223> primer for LEU4
<400> 13
   acggtggaag cattaacagg 20
<210> 14
   <211> 20
   <212> DNA
   <213> Artificial
<220>
   <223> primer for LEU4
<400> 14
   ggatccaatg gcaagtatgg 20
<210> 15
   <211> 20
   <212> DNA
   <213> Artificial
<220>
   <223> primer for OLE1
<400> 15
   ctccgttttc tactacgctg 20
<210> 16
   <211> 20
   <212> DNA
   <213> Artificial
<220>
   <223> primer for OLE1
<400> 16
   gttgggtcgt attggtacca 20
<210> 17
   <211> 20
   <212> DNA
   <213> Artificial
<220>
   <223> primer for SSU1
<400> 17
   tgctcttacg aggcagtttg 20
<210> 18
   <211> 20
   <212> DNA
   <213> Artificial
<220>
   <223> primer for SSU1
<400> 18
   atggcatgca gccacgttaa 20
<210> 19
   <211> 20
   <212> DNA
   <213> Artificial
<220>
   <223> primer for Lg-FLO1
<400> 19
   caacaaagca aaccaagggg 20
<210> 20
   <211> 20
   <212> DNA
   <213> Artificial
<220>
   <223> primer for Lg-FLO1
<400> 20
   ttaccatacg attgccagca 20
<210> 21
   <211> 18
   <212> DNA
   <213> Artificial
<220>
   <223> primer for 18s rRNA
<220>
   <221> misc_feature
   <222> (10)..(10)
   <223> y = t/u or c
<400> 21
   ctggttgaty ctgccagt 18
<210> 22
   <211> 18
   <212> DNA
   <213> Artificial
<220>
   <223> primer for 185 rRNA
<220>
   <221> misc_feature
   <222> (2)..(2)
   <223> y = t/u or c
<220>
   <221> misc_feature
   <222> (10)..(10)
   <223> r = g or a
<400> 22
   cygcaggttc acctacrg 18
<210> 23
   <211> 19
   <212> DNA
   <213> Artificial
<220>
   <223> primer for 28s rRNA
<220>
   <221> misc_feature
   <222> (1)..(1)
   <223> r = g or a
<220>
   <221> misc_feature
   <222> (17)..(17)
   <223> y = t/u or c
<220>
   <221> misc_feature
   <222> (18)..(18)
   <223> w = a or t/u
<400> 23
   rcatcgatga agaacgywg 19
<210> 24
   <211> 22
   <212> DNA
   <213> Artificial
<220>
   <223> primer for 28s rRNA
<220>
   <221> misc_feature
   <222> (1)..(1)
   <223> m = a or c
<220>
   <221> misc_feature
   <222> (2)..(2)
   <223> r = g or a
<220>
   <221> misc_feature
   <222> (7)..(7)
   <223> k = g or t/u
<220>
   <221> misc_feature
   <222> (15)..(15)
   <223> r = g or a
<220>
   <221> misc_feature
   <222> (16)..(16)
   <223> y = t/u or c
<220>
   <221> misc_feature
   <222> (17)..(17)
   <223> r = g or a
<400> 24
   mrggctkaat ctcaryrgat cg 22